(19) Europäisches Patentamt
European Patent Office
Office européen des brevets

(11) Veröffentlichungsnummer : **0 030 680**
**B1**

(12) # EUROPÄISCHE PATENTSCHRIFT

(45) Veröffentlichungstag der Patentschrift : **24.08.83**

(51) Int. Cl.³ : **A 61 K 7/13**, C 07 D249/18

(21) Anmeldenummer : **80107585.4**

(22) Anmeldetag : **04.12.80**

(54) **Haarfärbemittel.**

(30) Priorität : **13.12.79 DE 2950032**

(43) Veröffentlichungstag der Anmeldung : **24.06.81 Patentblatt 81/25**

(45) Bekanntmachung des Hinweises auf die Patenterteilung : **24.08.83 Patentblatt 83/34**

(84) Benannte Vertragsstaaten : **AT BE CH DE FR GB IT LI NL SE**

(56) Entgegenhaltungen :
**DE A 1 804 066**
**DE A 1 921 911**
**DE A 2 527 791**
**DE A 2 719 179**
**US A 2 334 348**
**US A 2 671 775**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Konrad, Günther, Dr.**
**Feuerbachweg 12**
**D-4010 Hilden (DE)**
Erfinder : **Lieske, Edgar**
**Hunsrückenstrasse 40**
**D-4000 Düsseldorf (DE)**

EP 0 030 680 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

« Haarfärbemittel »

Gegenstand der Erfindung sind Mittel zur oxidativen Färbung von Haaren auf Basis von substituierten Benzotriazolen als Kupplerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, daß auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gar Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß Haarfärbemittel auf Basis von Oxidationsfarbstoffen mit einem Gehalt an substituierten Benzotriazolen der Formel

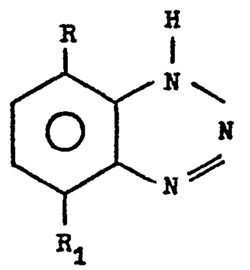

in der R und $R_1$ Wasserstoff oder eine Aminogruppe darstellen, mit der Maßgabe, daß wenigstens einer der Reste R oder $R_1$ eine Aminogruppe ist und deren Addukten mit 1-4 Mol Ethylenoxid pro Mol substituierten Benzotriazols, sowie deren anorganischen oder organischen Salzen als Kupplersubstanzen und den in Oxidationshaarfarben üblichen Entwicklerkomponenten den gestellten Anforderungen weitgehend gerecht werden.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, unterschiedliche, von dunkelblond über braun bis braunviolett reichende Farbtöne und stellen somit eine wesentliche Bereicherung der oxidativen Haarfärbemöglichkeiten dar. Darüber hinaus zeichnen sich die erfindungsgemäß einzusetzenden substituierten Benzotriazole durch sehr gute Echtheitseigenschaften der damit erzielten Färbungen, durch eine gute Löslichkeit in Wasser und eine gute Lagerstabilität aus.

Die erfindungsgemäß als Kupplerkomponenten zu verwendenden substituierten Benzotriazole können entweder als solche oder in Form ihrer Salze mit anorganischen oder organischen Säuren, wie zum Beispiel als Chloride, Sulfate, Phosphate, Acetate, Propionate, Lactate, Citrate eingesetzt werden.

Die erfindungsgemäß als Kupplerkomponenten zu verwendenden substituierten Benzotriazole stellen literaturbekannte Verbindungen dar, deren Herstellung von K. Fries, H. Güterbock und H. Kuhn in der Veröffentlichung « Untersuchungen in der Reihe des Azimidobenzols » in Annalen der Chemie 511 (1934) Seite 229 sowie von S. Angeloni et al. in Tetrahedron 28 (1972) Seite 313 beschrieben wird, ohne deren Eignung als Farbstoffkomponente für Haarfarbstoffe anzusprechen. Die Herstellung der Addukte mit 1-4 Mol Ethylenoxid erfolgt nach den allgemein bekannten Verfahren der Ethoxylierung von Verbindungen mit aktiven Wasserstoffatomen durch Umsetzung der Aminobenzotriazolderivate mit Ethylenoxid im Autoklaven bei 100 °C.

Als erfindungsgemäß zu verwendende Kupplerkomponenten sind 4(7)-Aminobenzotriazol, 4,7-Diaminobenzotriazol, 4(7)-Aminobenzotriazol + 1 Ethylenoxid, 4(7)-Aminobenzotriazol + 2 Ethylenoxid, 4(7)-Aminobenzotriazol + 3 Mol Ethylenoxid, 4(7)-Aminobenzotriazol + 4 Mol Ethylenoxid, 4,7-Diaminobenzotriazol + 1 Mol Ethylenoxid, 4,7-Diaminobenzotriazol + 2 Mol Ethylenoxid, 4,7-Diaminobenzotriazol + 3 Mol Ethylenoxid, 4,7-Diaminobenzotriazol + 4 Mol Ethylenoxid zu nennen.

Als Beispiele für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxy-ethylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate wie 1-Methyl-pyrrolidon (2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino-6-piperidino-pyrimidin, 2,4,5-Triamino-6-anilino-pyrimidin, 2,4,5-Triamino-6-morpholinopyrimidin, 2,4,5-Triamino-6-β-hydroxy-ethylamino-pyrimidin anzuführen.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden substituierten Benzotriazole darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

## Beispiele

Als erfindungsgemäß zu verwendende substituierte Benzotriazole wurden folgende Produkte in den nachfolgend genannten Beispielen als Kupplersubstanzen eingesetzt:

A) 4(7)-Aminobenzotriazol (Gemisch der tautomeren Formen)
B) 4,7-Diaminobenzotriazol
C) 4(7)-Aminobenzotriazol + 1 Ethylenoxid
D) 4(7)-Aminobenzotriazol + 2 Ethylenoxid
E) 4(7)-Aminobenzotriazol + 4 Ethylenoxid

Die Ethylenoxidaddukte wurden durch 6 Stunden langes Erhitzen von 4(7)-Aminobenzotriazol mit den jeweiligen Mengen Ethylenoxid in Ethanol im Autoklaven auf 100 °C erhalten. Die Verbindungen stellen dunkelbraune Öle dar.

Als Entwicklerkomponenten dienten folgende Verbindungen :

E1) 2,4,5,6-Tetraaminopyrimidin
E2) p-Toluylendiamin
E3) N,N-Bis-(2-hydroxyethylamino)-m-phenylendiamin
E4) 2-Methylamino-4,5,6-triaminopyrimidin
E5) 1-Phenyl-3-carbamoyl-4-aminopyrazolon
E6) 2,5-Diaminoanisol
E7) 2-Chlor-p-phenylendiamin.

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,
10 Gewichtsteilen Fettalkoholsulfat (Natriumsalz) Kettenlänge $C_{12}$-$C_{18}$,
75 Gewichtsteilen Wasser

jeweils 0,01 Mol der in der nachstehenden Tabelle aufgeführten Entwicklersubstanzen und substituierten Benzotriazole eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit oder ohne zusätzlichem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | a) Entwickler | b) Kuppler | Erhaltener Farbton mit 1 %iger $H_2O_2$-Lösung |
|---|---|---|---|
| 1 | E 1 | A | graubraun |
| 2 | E 2 | A | dunkelviolett |
| 3 | E 1 | B | rehbraun |
| 4 | E 2 | B | purpurgrau |
| 5 | E 1 | C | hellbraun |
| 6 | E 2 | C | braunviolett |
| 7 | E 3 | C | blaugrau |
| 8 | E 1 | D | goldbraun |
| 9 | E 2 | D | dunkelrotbraun |
| 10 | E 3 | D | blaugrau |
| 11 | E 6 | D | blaugrau |
| 12 | E 7 | D | graubraun |
| 13 | E 4 | D | nougatfarbig |
| 14 | E 1 | E | dunkelblond |
| 15 | E 2 | E | dunkelbraun |
| 16 | E 3 | E | blaugrau |
| 17 | E 4 | E | graubraun |
| 18 | E 5 | E | braungrau |

**Ansprüche**

1. Haarfärbemittel auf Basis von Oxidationsfarbstoffen, gekennzeichnet durch einen Gehalt an substituierten Benzotriazolen der Formel

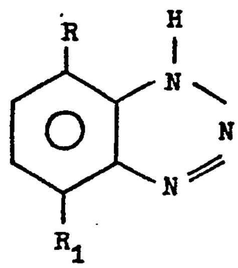

in der R und $R_1$ Wasserstoff oder eine Aminogruppe darstellen, mit der Maßgabe, daß wenigstens einer der Reste R oder $R_1$ eine Aminogruppe ist und deren Addukten mit 1-4 Mol Ethylenoxid pro Mol substituierten Benzotriazols, sowie deren anorganischen oder organischen Salzen als Kupplersubstanzen und den in Oxidationshaarfarben üblichen Entwicklerkomponenten.

2. Haarfärbemittel nach Anspruch 1, gekennzeichnet durch einen Gehalt weiterer üblicher Kupplersubstanzen sowie gegebenenfalls üblicher direktziehender Farbstoffe.

3. Haarfärbemittel nach Anspruch 1 und 2, gekennzeichnet durch einen Gehalt an Entwickler-Kuppler-Kombination von 0,2 bis 5 Gewichtsprozent, vorzugsweise von 1 bis 3 Gewichtsprozent, bezogen auf das gesamte Mittel.

**Claims**

1. Hair dye compositions based on oxidation dyes, characterised in that they contain substituted benzotriazoles corresponding to the following formula

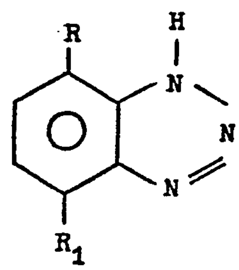

in which R and $R_1$ represent hydrogen or an amino group, with the proviso that at least one of the radicals R or $R_1$ is an amino group, and their adducts with 1 to 4 moles of ethylene oxide per mole of substituted benzotriazole and also their inorganic or organic salts as coupler substances and the developer components normally used in oxidation hair dyes.

2. Hair dye compositions as claimed in Claim 1, characterised in that they contain other standard coupler substances and optionally standard substantive dyes.

3. Hair dye compositions as claimed in Claims 1 and 2, characterised in that they contain a combination of developer and coupler in a quantity of from 0.2 to 5 % by weight and preferably in a quantity of from 1 to 3 % by weight, based on the composition as a whole.

**Revendications**

1. Agent de teinture pour cheveux à base de colorants d'oxydation, caractérisé en ce qu'il contient des benzotriazoles substitués de formule

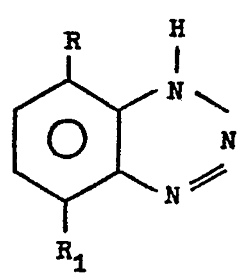

dans laquelle R et $R_1$ représentent chacun un atome d'hydrogène ou un groupe amino, à condition qu'au moins un des radicaux R et $R_1$ soit un groupe amino, ainsi que leurs produits d'addition avec 1-4 moles d'oxyde d'éthylène par mole du benzotriazole substitué, ainsi que leurs sels inorganiques ou organiques comme substances copulantes et composants révélateurs habituellement utilisés dans la teinture des cheveux par oxydation.

2. Agent de teinture pour cheveux suivant la revendication 1, caractérisé en ce qu'il contient des substances copulantes habituelles supplémentaires, ainsi qu'éventuellement des colorants habituels à fixation directe.

3. Agent de teinture pour cheveux suivant les revendications 1 et 2, caractérisé en ce qu'il contient une combinaison révélateur/copulant en une teneur de 0,2 à 5 % en poids, de préférence, de 1 à 3 % en poids, calculé sur la totalité de l'agent de teinture pour cheveux.